# EUROPEAN PATENT SPECIFICATION

(11) **EP 0 748 319 B1**
(45) Date of publication and mention of the grant of the patent: **12.12.2001**
(21) Application number: 95911105.5
(22) Date of filing: 27.02.1995
(51) Int. Cl.: C07D 413/06, C07D 263/52, C07D 413/04

(54) **PROCESS FOR MAKING AN EPOXIDE**
VERFAHREN ZUR HERSTELLUNG VON EPOXIDEN
PROCEDE DE FABRICATION D'UN EPOXYDE

(30) Priority: 04.03.1994 US 206074
(43) Date of publication of application: 18.12.1996
(73) Proprietor: Merck & Co., Inc., Rahway New Jersey 07065-0900 (US)
(72) Inventor: ASKIN, David, Rahway, NJ 07065 (US); ENG, Kan, K., Rahway, NJ 07065 (US); MALIGRES, Peter, E., Rahway, NJ 07065 (US); REIDER, Paul, J., Rahway, NJ 07065 (US); ROSSEN, Kai, Rahway, NJ 07065 (US); VOLANTE, Ralph, P., Rahway, NJ 07065 (US); UPADHYAY, Veena, Rahway, NJ 07065 (US)
(74) Representative: Cole, William Gwyn
(86) International application number: US9502347
(87) International publication number: WO9523797

(56) References cited:
- EP-A- 0 521 686
- EP-A- 0 541 168
- TETRAHEDRON LETTERS, vol. 35,no. 5, 31 January 1994 OXFORD GB, pages 673-676, DAVID ASKIN ET AL 'Highly diastereoselective reaction of a chiral,non-racemic amide enolate with (s)-glycidyl tosylate.Synthesis of the orally active HIV-1 protease inhibitor L-735,524 '

## Description

### BACKGROUND OF THE INVENTION

This case is related to Merck Cases US 5,618,939, US 5,491,238, US 5,463,063 and U.S. Patent 5,169,952.

The present invention is concerned with a novel intermediate and process for synthesizing compounds which inhibit the protease encoded by human immunodeficiency virus (HIV), and in particular, the compound disclosed and referred to as "Compound J" in EPO 541,168, which published on May 12, 1993, or pharmaceutically acceptable salts thereof. 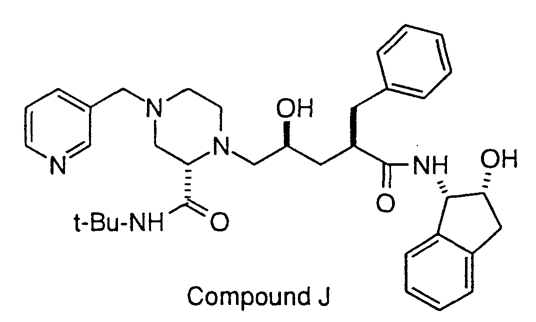 These compounds are of value in the prevention of infection by HIV, the treatment of infection by HIV and the treatment of the resulting acquired immune deficiency syndrome (AIDS).

More specifically, the instant process involves the preparation of the epoxide intermediate for the production of Compound J, the HIV protease inhibitor depicted above. The process relates to iodohydrin formation of the allyl acetonide via the intermediate iodoiminolactone. Base-induced cyclization of the iodohydrin then forms the epoxide intermediate. The iodohydrin formation proceeds with high diastereoselectivity, and there is a substantial absence of hydrolysis of the amide bond linkage in this process.

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E. *et al*., *Proc. Nat'l Acad. Sci*., *85,* 4686 (1988), demonstrated that genetic inactivation of the HIV encoded protease resulted in the production of immature, non-infectious virus particles. These results indicate that inhibition of the HIV protease represents a viable method for the treatment of AIDS and the prevention or treatment of infection by HIV.

The nucleotide sequence of HIV shows the presence of a pol gene in one open reading frame [Ratner, L. *et al., Nature, 313,* 277 (1985)]. Amino acid sequence homology provides evidence that the pol sequence encodes reverse transcriptase, an endonuclease and an HIV protease [Toh, H. *et al., EMBO J., 4,* 1267 (1985); Power, M.D. *et al., Science, 231,* 1567 (1986); Pearl, L.H. *et al., Nature, 329,* 351 (1987)]. The end product compounds, including Compound J, that can be made from the novel intermediates and process of this invention are inhibitors of HIV protease, and are disclosed in EPO 541,168, which published on May 12, 1993.

Previously, the synthesis of Compound J and related compounds was accomplished via a 12-step procedure which employed a hydroxy protected dihydro-5(S)-hydroxymethyl-3(2H) furanone which was alkylated, and involved replacement of an alcohol leaving group on the alkylated furanone with a piperidine moiety. The coupled product was then hydrolyzed to open the furanone ring into a hydroxy acid moiety, and the acid was ultimately coupled to 2(R)-hydroxy-1(S)aminoindane. This procedure is described in EPO 541,168. The extreme length of this route (12 steps), renders this process time consuming and labor intensive, and it requires the use of many expensive reagents and an expensive starting material. A route requiring fewer reaction steps and/or more efficient reagents would provide desirable economical and timesaving benefits.

The iodolactamization of olefinic tertiary amide **A** is known to occur with subsequent hydrolysis of the charged iodoiminolactam **B** intermediate giving the iodolactone **C** as the only isolated product (Scheme ALPHA). See Tamaru, Y. *et al., J. Am. Chem. Soc., 106*, 1079-1085 (1984); Trost, B.M. *et al*., eds. *Comprehensive Organic Synthesis; Selectivity, Strategy, & Efficiency in Modern Organic Chemistry, Volume 4*, Pergamon Press, New York 1991, p. 398-421. In this process, it is known that very efficient chirality transfer occurs from the 2-position to the 4-position, to give the 2,4-syn products (represented by the corresponding hydroxy-acid **D**) in high diastereoselectivity.

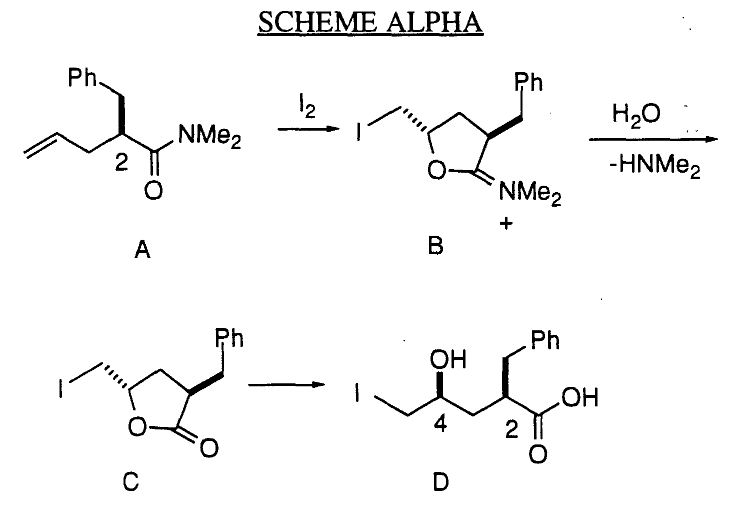

In another existing process, the acetonide is reacted with (S)glycidyl tosylate in the presence of strong base LHMDS to form the epoxide (see Scheme BETA). Since both the starting material (S)glycidyl tosylate and product are epoxides, the acetonide anion reacts also with the product epoxide; therefore, about 20% double addition byproducts after formed, in addition to the product epoxide in 71 % yield. After crystallization from MeOH, an additional MTBE recrystallization was required to provide the epoxide free of dimer; consequently the overall isolated yield from the acetonide can range from 56-61%. The formation of double nucleophilic addition products is a problem inherent to the electrophile glycidyl tosylate. The (S)-glycidyl tosylate is also presently the most costly raw material in the synthesis of Compound J.

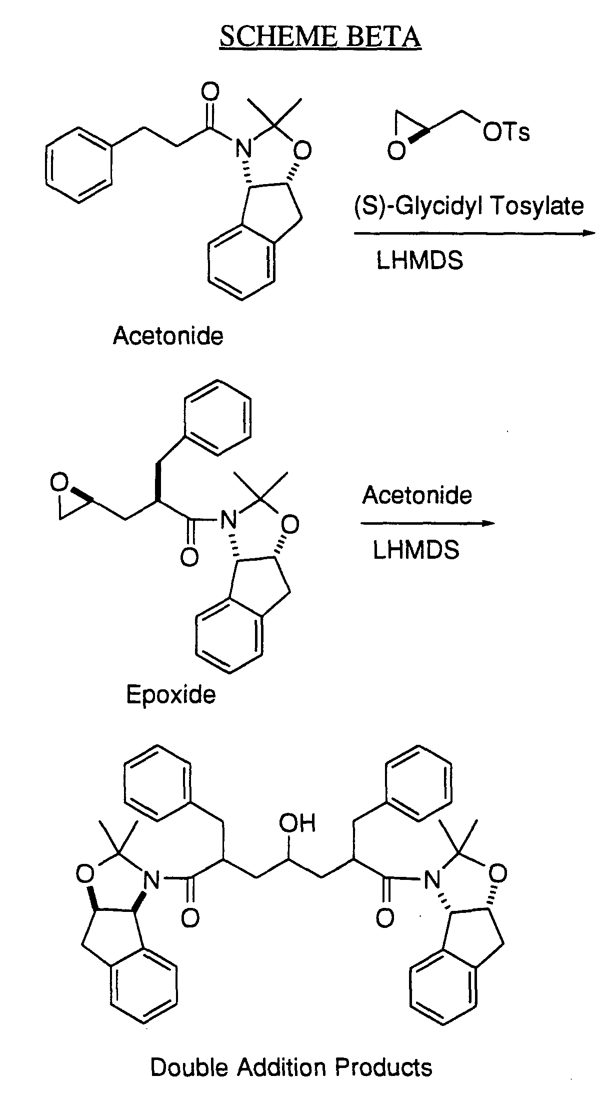

The reaction of the lithium enolate of the acetonide intermediate with electrophiles is known to occur with very high selectivity and in high yield for the desired 2(R) products. See Askin, D. *et al., J. Org. Chem., 57,* 2771-2773 (1992); Askin, D. *et al., Tetrahedron Lett.., 35,* 673-676 (1994). It was also known that halohydrin derivatives are cleanly converted to the desired epoxide intermediate for Compound J. It was however unexpected that the conditions of the instant invention would result in the isolation of the iodohydrin in excellent yield, exceeding 70% overall from the acetonide intermediates.See also, EPO 541168, EPO 521686 and Tamasa, Y. et al., J. Am. Chem. Soc., 106, 1079 (1984).

### SUMMARY OF THE INVENTION

A process is disclosed for synthesizing the epoxide of the formula 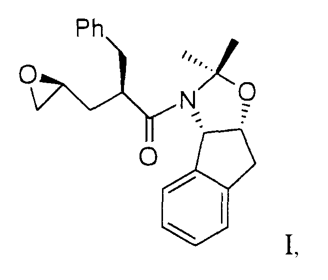 comprising the first step of formation of a halohydrin from the allyl acetonide reactant, followed by the step of base-induced cyclization. An additional first step involves allylation of an acetonide reactant to form the allyl acetonide. The products are useful as intermediates for the synthesis of inhibitors of renin or HIV protease or other proteases.

| ABBREVIATIONS | |
|---|---|
| Designation | Protecting Group |
| BOC (Boc) | t-butyloxycarbonyl |
| CBZ (Cbz) | benzyloxycarbonyl (carbobenzoxy) |
| TBS (TBDMS) | t-butyl-dimethylsilyl |

| Designation | Activating Group |
|---|---|
| Ts or tosyl or tosylate | p-toluenesulfonyl |
| Ns or nosyl or nosylate | 3-nitrobenzenesulfonyl |
| Tf or triflyl or triflate | trifluoromethanesulfonyl |
| Ms or mesyl or mesylate | methanesulfonyl |

| Designation | Coupling Reagent |
|---|---|
| BOP reagent | benzotriazol-1-yloxytris(dimethylamino)phosphonium hexafluorophosphate |
| BOP-Cl | bis(2-oxo-3-oxazolidinyl)phosphinic chloride |
| EDC | 1-ethyl-3-(3-dimethylaminopropyl) carbodiimide hydrochloride |

| | Other |
|---|---|
| BOC-ON | 2-(tert-butylcarbonyloxyimino)-2-phenylacetonitrile |
| (BOC)₂O (BOC₂O or Boc₂O) | di-t-butyl dicarbonate |
| n-Bu₄N⁺F⁻ | tetrabutyl ammonium fluoride |
| nBuLi (n-Buli) | n-butyllithium |
| (S)-CSA | (1*S*)-(+)-10-camphorsulfonic acid |
| DABCO | diazabicyclooctane |
| DBN | diazabicyclononane |
| DBU | diazabicyloundecane |
| DI | deionized |
| DIEA or DIPEA | diisopropylethylamine |
| DMA | dimethylacetamide |
| DMAP | dimethylaminopyridine |
| DME | dimethoxyethane |
| DMF | dimethylformamide |
| DMPU | dimethyltetrahydropyrimidinone |
| DMSO | dimethylsulfoxide |
| Et3N | triethylamine |
| EtOAc | ethyl acetate |
| h | hour(s) |
| IPA | 2-propanol |
| KF | Karl Fisher titration for water |
| LDA | lithium diisopropylamide |
| LHMDS | lithium hexamethyldisilazide |
| L-PGA | (L)-pyroglutamic acid |
| MeCN | acetonitrile |
| MTBE | methyl t-butyl ether |
| NMP | N-methylpyrrolidinone |
| r.t. | room temperature |
| TFA | trifluoroacetic acid |
| TG | thermal gravimetry: loss on heating |
| THF | tetrahydrofuran |
| TLC | thin layer chromatography |
| TMEDA | tetramethylethylenediamine |
| TMU | tetramethyl urea |

### DETAILED DESCRIPTION OF THE INVENTION

The process of the present invention is illustrated by the following Scheme:

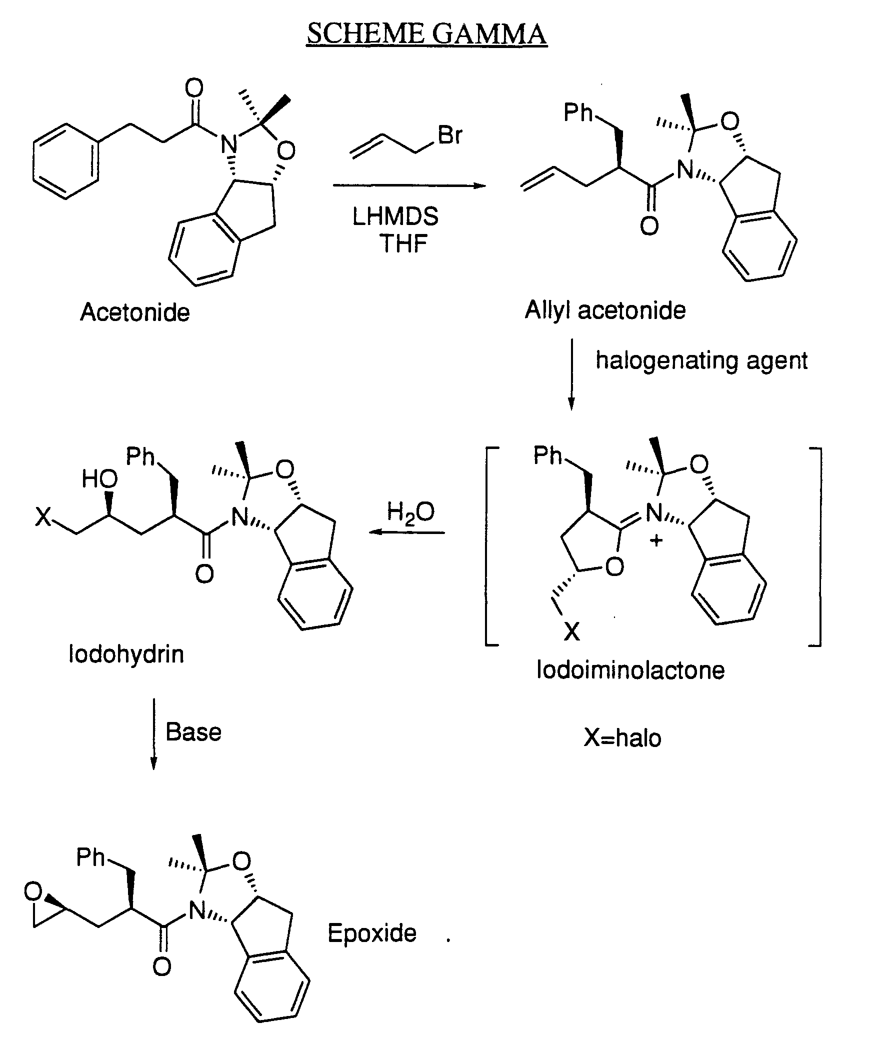

The above scheme illustrates three steps, beginning with allylation of the acetonide, followed by halohydrin formation, and then base-induced cyclization forming product epoxide.

In the present invention, a process of synthesizing the epoxide of Formula I, 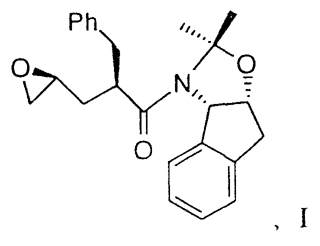 comprises the steps of:
(a) contacting one equivalent of the allyl acetonide of Formula II, 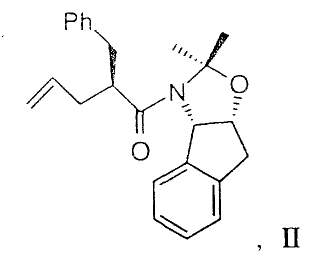 with one to two equivalents of a halogenating agent in solvent mixed with aqueous weak base, at a temperature range of between -40°C and 100°C, to form the halohydrin of Formula III, and 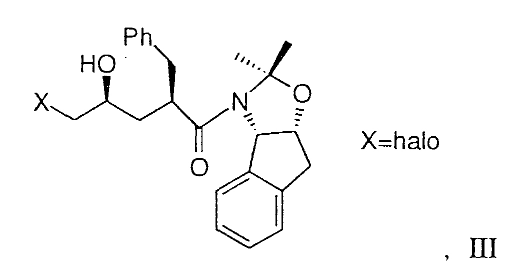
(b) adding base in solvent or solvents to elicit the formation of the epoxide of Formula **I**.

From the acetonide reactant further comprises
reacting one equivalent of the acetonide 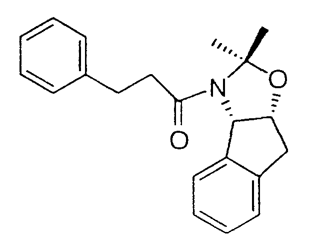 with one equivalent of allylhalide in strong base, to give the allyl acetonide of Formula **II**.

In the preparation of the allyl acetonide of Scheme GAMMA, the preferred allylation reagents include allyl halides such as allyl chloride, allyl bromide or allyl iodide, as well as other allyl electrophiles such as allyl methane sulfonate or allyl esters in the presence of a transition metal catalyst. Most preferred allylation reagents include allyl halides such as allyl chloride, allyl bromide, and allyl iodide.

For this allylation reaction, preferred bases are strong bases and include amide bases such as the lithium, sodium, potassium or magnesium salts of amines, such as diethylamine, disopropylamine, dicyclohexylamine, piperidine, pyrrolidine, or bistrimethylsilylamine; metal alkyls such as the C₁₋₆ alkyllithium such as n-, iso-, sec-, and tert-butyllithium, methyl, ethyl, or aryl lithiums such as phenyllithium; Grignard reagents such as methyl ethyl, propyl, or butyl magnesium halide; alkoxides such as the methoxide, ethoxide, isopropoxide, tert-butoxide, tert-amyloxide alkoxides of lithium, sodium, potassium or magnesium.

In the allylation reaction, the most preferred base is lithium hexamethyldisilazide (LHMDS).

Also in the allylation reaction, preferred solvents include ethereal solvents such as THF, DME, MTBE, diethylether, diglyme, or dibutylether; hydrocarbon solvents such as pentane, hexane, heptane, benzene, toluene or ethyl benzene; or other solvents compatible with bases and organometallic reagents such as DMSO, DMPU, NMP, TMU, TMEDA, and crown ethers; and including mixtures of these solvents.

Most preferred solvents for allylation are ethereal solvents for allylation such as THF, DME, and MTBE.

The preferred temperature range for the allylation is -78°C to +30°C. The incubation period lasts at least 15 minutes and typically up to 3 hours.

For halohydrin formation, preferred halogenating reagents include halogens, interhalogen compounds, halonium salts, or hypohalite salts or esters, oxyhalogen salts or acids, halo-amides, halo-ureas, halo-carbamates, halo-sulfonamides, halo-amines, or other halogenated nitrogen compounds, or combinations thereof with either halide salts or phase transfer catalysts or both. Preferred halogenating reagents are hypohalite salts or esters, halo amides, ureas, carbamates, sulfonamides, amines, or other halogenated nitrogen compounds such as N-iodosuccinimide, N-bromosuccinimide with an iodide salt, or N-chlorosuccinimide with an iodide salt. Most preferred halogenating reagents are N-iodosuccinimide, N-bromosuccinimide in combination with an iodide salt, or N-chlorosuccinimide in combination with an iodide salt.

Reaction conditions for halohydrin formation are solutions, suspensions, or other biphasic systems containing weak bases such as sodium bicarbonate, calcium carbonate, magnesium hydroxide, basic alumina, neutral alumina, sodium acetate, dibasic sodium phosphate, dibasic potassium phosphate, potassium fluoride, other salts, or water in common organic solvents. Preferred reaction conditions are weak bases such as sodium bicarbonate, basic alumina, potassium fluoride, or water. Most preferred reaction conditions are basic alumina, or sodium bicarbonate. Solvents must be compatible with the reaction conditions and include ethers, aromatic chlorinated hydrocarbons, esters, alcohols, MeCN, DMF, DMPU, or ketones. Preferred are chlorinated hydrocarbons, ethers and esters. Most preferred are dichloromethane, IPAC, EtOAc, DME, and MTBE. Temperature range is between -40°C and 100°C, but preferably between 0 and 35°C. Incubation lasts at least 10 minutes and is typically stopped before 48 hours.

Base-induced cyclization to form the epoxide is accomplished by treating the halohydrin with a base. Preferred bases for such cyclization include hydroxides and oxides of lithium, sodium, potassium, magnesium, calcium, or tetraalkylammonium; alkoxides such as lithium, sodium, potassium, magnesium, and tetraalkylammonium methoxide, ethoxide, n- and iso-propoxide, n-, iso-, sec-, and tert-butoxide. Other suitable bases include tertiary and hindered amines such as triethylamine, DIEA, DBU, DBN, DABCO, methyl morpholine, diisiopropylamine, dicyclohexyl amine, bis trimethyl-silylamine or tetramethylpiperidine as well as metal amide salts thereof. Most preferred bases are lithium, sodium, potassium, or tetraalkylammonium hydroxides; alkoxides such as lithium, sodium and potassium methoxide, ethoxide, iso-propoxide, or tert-butoxide; or tertiary amines such as DIEA. Alkali hydroxide means LiOH, KOH, or NaOH or mixtures thereof.

Also for the base-induced cyclization, preferred solvents are ethers, esters, hydrocarbons, aromatic solvents, chlorinated hydrocarbons, ketones, water, alcohols, DMSO, MECN, DMF, or DMPU, or other polar solvents, or mixtures thereof. Most preferred solvents are ethers, esters, alcohols, or polar aprotic solvents.

Base-induced cyclization is carried out in a temperature range of between -40°C and 100°C. Incubation lasts at least 10 minutes and is typically stopped before 48 hours.

In the process of the present invention, a wide variety of solvents can be used, except where noted. Hydrocarbon solvents include pentane, hexane, heptane, cyclohexane, methyl-cyclohexane, benzene, toluene and xylene. Aromatics as solvents include benzene, toluene, xylene, and ethyl-benzene. Chlorinated hydrocarbons as solvents include methylene chloride, chloroform, carbontetrachloride, dichloroethane, trichloroethane, tetrachloroethane, trichloroethylene, tetrachloroethylene, chlorobenzene and dichlorobenzene. Ethers as solvents include diethyl ether, dibutylether, tetrahydrofuran, dimethoxyethane, diethoxyethane, and MTBE. Esters as solvents include ethyl acetate, IPAC, and ethoxyethyl acetate. Ketones as solvents include acetone, MEK, and MIBK. Alcohols as solvents include methanol, ethanol, propanol, isopropanol, butanol, and methoxyethanol. Polar aprotic solvents as solvents include DMF, DMA, DMSO, DMPU, TMU, NMP and acetonitrile. Tertiary amines as solvents include triethylamine, diisopropyl ethyl amine, pyridine, DABCO, DBU, DBN, pentamethyl piperidine, and DMAP.

In one embodiment of the present invention, a process of synthesizing the epoxide of Formula **I**, 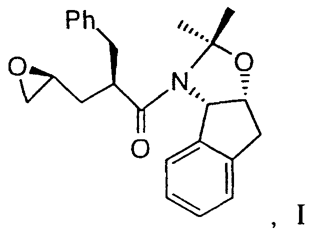 comprises the steps of:
(a) contacting one equivalent of the allyl acetonide of Formula **II,** 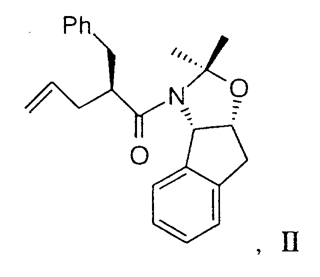 with one to two equivalents of a halogenating agent, in solvent mixed with aqueous weak base, at a temperature range of between -40°C and 100°C, to form the halohydrin of Formula III, 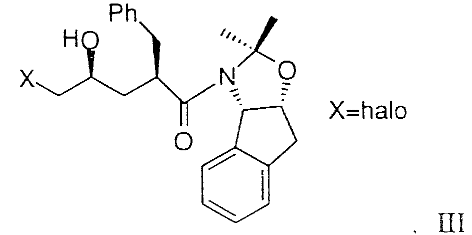 said halogenating agent selected from N-iodosuccinimide, N-bromosuccinimide or N-chlorosuccinimide, the last two of which may be combined with an iodide salt, said solvent selected from the group consisting of dichloromethane, IPAC, EtOAc, DME, and MTBE, said weak base selected from basic alumina or sodium bicarbonate, and
(b) adding base in water to elicit formation of the epoxide of Formula I, said base selected from lithium hydroxide, sodium hydroxide, potassium hydroxide, tetralkylammonium hydroxide; any C₁₋₄ alkoxide of lithium, sodium or potassium; and DIEA.

In another embodiment of the present invention involving the acetonide reactant, the present process of synthesizing the epoxide of Formula **I**, 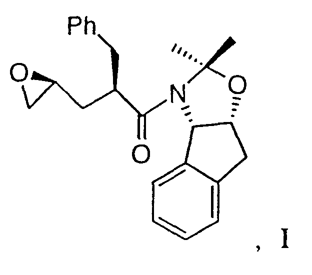 further comprises the step of
reacting one equivalent of the acetonide 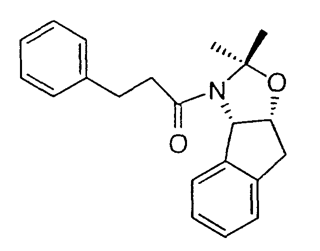 with one equivalent of allylhalide in strong base, said allyl halide selected from allyl chloride, allyl bromide and allyl iodide, to give the allyl acetonide of Formula **II**.

In another embodiment of the present invention, a process of synthesizing the epoxide of Formula **I**, 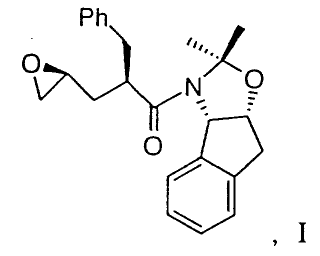 comprises the steps of:
(a) contacting one equivalent of the allyl acetonide of Formula **II**, 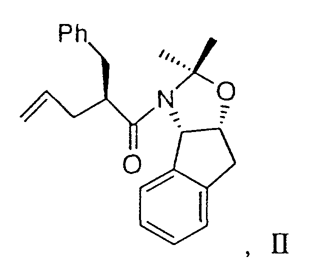 said allyl acetonide dissolved in isopropyl acetate, with one to two equivalents of N-iodosuccinimide in about 0.5 M aqueous sodium bicarbonate, at room temperature, to form the iodohydrin of Formula **III**, and 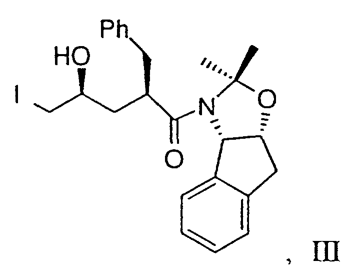
(b) adding alkali hydoxide in water to elicit formation of the epoxide of Formula **I**.

In another embodiment of the present invention, the process of synthesizing the epoxide of Formula **I**, 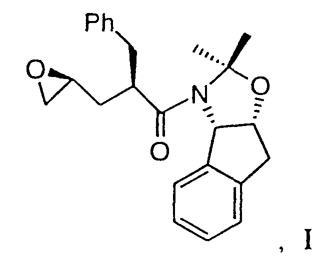 comprises the steps of:
(a) reacting one equivalent of the acetonide 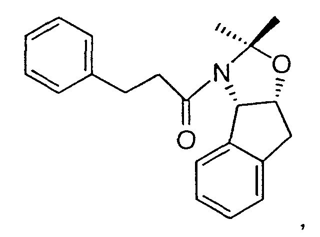 said acetonide dissolved in ethereal solvent, with one equivalent of allylbromide and one equivalent of about 1.0-2.0 M lithiumhexamethyldisilazide (in ethereal solvent), to give the allyl acetonide of Formula **II**, 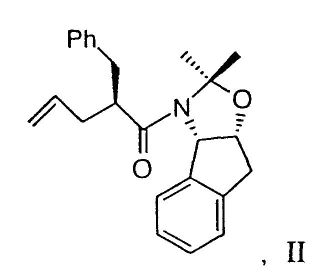
(b) mixing thereto one to two equivalents of N-iodosuccinimide in about 0.5 M aqueous sodium bicarbonate, at room temperature, to form the iodohydrin of Formula **III**, and 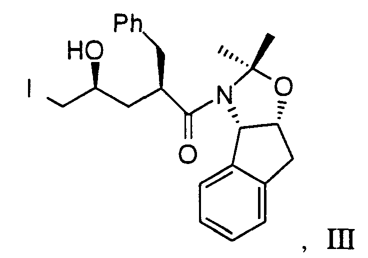
(c) adding alkali hydroxide in water to elicit formation of the epoxide of Formula **I**.

The processes and intermediates of this invention are useful for the preparation of end-product compounds that are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the end-product compounds that can be made from the processes and intermediates of this invention are useful in treating infection by HIV after suspected past exposure to HIV by, e.g., blood transfusion, organ transplant, exchange of body fluids, bites, accidental needle stick, or exposure to patient blood during surgery.

The end-product HIV protease inhibitors are also useful in the preparation and execution of screening assays for antiviral compounds. For example, end-product compounds are useful for isolating enzyme mutants, which are excellent screening tools for more powerful antiviral compounds. Furthermore, such compounds are useful in establishing or determining the binding site of other antivirals to HIV protease, e.g., by competitive inhibition. Thus the end-product compounds that are made from the processes and intermediates of this invention are commercial products to be sold for these purposes.

HIV protease inhibitor compounds that can be made from the intermediates and processes of the instant invention are disclosed in EPO 541,164. The HIV protease inhibitory compounds may be administered to patients in need of such treatment in pharmaceutical compositions comprising a pharmaceutical carrier and therapeutically-effective amounts of the compound or a pharmaceutically acceptable salt thereof. EPO 541,164 discloses suitable pharmaceutical formulations, administration routes, salt forms and dosages for the compounds.

The compounds of the present invention, may have asymmetric centers and occur as racemates, racemic mixtures and as individual diastereomers, or enantiomers with all isomeric forms being included in the present invention.

When any variable (e.g., aryl, heterocycle, R, R¹, R², n, X, etc.) occurs more than one time in any constituent, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl; t-Bu is tert-butyl); "Halo", as used herein, means fluoro, chloro, bromo and iodo. As used herein, "aryl" is intended to mean phenyl (Ph) or naphthyl.

Representative experimental procedures utilizing the novel process are detailed below. These procedures are exemplary only and should not be construed as being limitations on the novel process of this invention.

### EXAMPLE 1

### Conversion of Acetonide to Allyl Acetonide

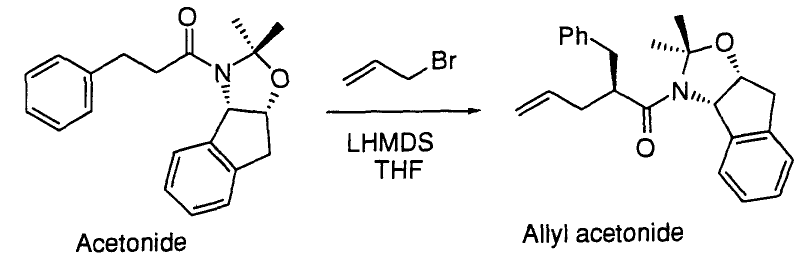

| | |
|---|---|
| Acetonide | 32.1 g |
| Allyl bromide | 12.70 g |
| Lithiumhexamethyldisilazide (LHMDS) 1.0 M in THF | 105 mL |
| Tetrahydrofuran (THF) | 200 mL |

The acetonide was dissolved in 200 mL THF in a 100 mL 3 neck flask equipped with an addition funnel and degassed by bubbling in nitrogen for 20 min. The mixture was cooled to -25°C and the allyl bromide was added via a weighed syringe. The LHMDS was transferred to the addition funnel under nitrogen pressure via cannula. The LHMDS was allowed to slowly drop into the magnetically stirred reaction mixture over 20 min. The internal temperature reached -14°C while the cooling bath was at -30°C. The mixture was aged at -20 to -15°C for 30 min. Water (100 mL) and IPAC (100 mL) were added and the temperature rose to 5°C. The lower aqueous phase was discarded and the organic phase was washed with 100 mL of 0.2 M HCl in 3% aq. NaCl, 30 mL brine, and 30 mL 0.5 M sodium bicarbonate. The organic phase was evaporated (55°C, 100 Torr) to an oil, another 40 mL of IPAC were added, and the mixture was again evaporated to an oil. At this point the crude allyl acetonide may be taken directly on to the next step or purified by crystallization from 30:1 hexane-IPAC or 30:1 methylcyclohexane-IPAC to give the allyl acetonide as a white crystalline solid in 87% yield.

| Allyl acetonide ¹³C NMR data for major rotamer (62.5 MHz) | | | |
|---|---|---|---|
| 171.0 | 140.4 | 140.2 | 134.8 |
| 129.6 | 128.6 | 128.2 | 127.1 |
| 126.6 | 125.6 | 124.0 | 117.9 |
| 96.8 | 78.9 | | |
| | 65.6 | 47.5 | 38.6 |
| 38.0 | 36.1 | 26.6 | 24.1 ppm |

### EXAMPLE 2

### Conversion of Allyl Acetonide to Iodohyrin and Cyclization to Epoxide with NIS

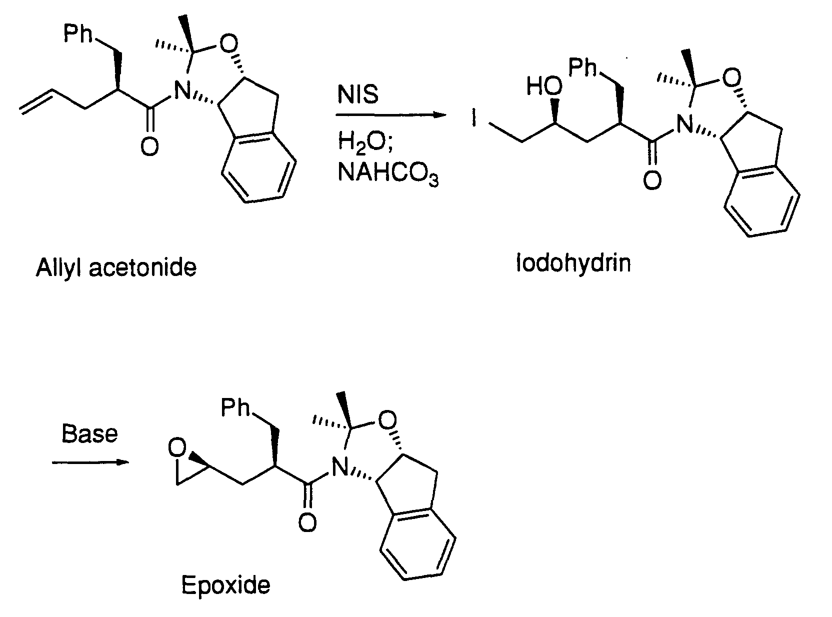

| | |
|---|---|
| Allyl acetonide (crude from above preparation) | ca 0.1 mol |
| N-iodosuccinimide (NIS) | 29.24 g |
| Aqueous sodium bicarbonate (0.5 M) | 350 mL |
| Isopropyl acetate (IPAC) | 300 mL |

The crude allyl acetonide was dissolved in IPAC and stirred with the aqueous sodium bicarbonate and NIS for 17 h. Aqueous sodium bisulfite (38-40%) solution was added and the upper organic phase was separated. The organic phase was washed with 300 mL water and 2 x 100 mL brine. At this point the crude iodohydrin solution in IPAC can be directly taken on to the next step or the solution could be evaporated and crystallized from methylcyclohexane-IPAC to give the iodohydrin as a pale yellow crystalline solid, ¹³C NMR:, m.p. rotation.

| | |
|---|---|
| Iodohydrin (IPAC solution crude from above preparation) | ca 0.1 mol |
| Lithium hydroxide monohydrate | 50 g |
| Water | 200 mL |

| Iodohydrin ¹³C NMR data for major rotamer (62.5 MHz) | | | |
|---|---|---|---|
| 172.2 | 140.6 | 140.4 | 139.3 |
| 129.5 | 128.8 | 128.2 | 127.2 |
| 126.8 | 125.7 | 124.0 | 96.9 |
| 79.1 | 68.7 | 65.8 | 43.7 |
| 40.6 | 39.0 | 36.2 | 26.5 |
| 24.3 | 16.3 ppm | | |

### EXAMPLE 3

### Conversion of Allyl Acetonide to Iodohyrin and Cyclization to Epoxide with NCS/NaI

The iodohydrin in IPAC was stirred with the lithium hydroxide in water for 3 h at 25-30°C. The upper organic phase was washed with 200 mL water and 200 mL brine and was dried over ca 2 g of magnesium sulfate. The IPAC solution was filtered and evaporated (50-60°C, 100 Torr) down to ca 50 mL when the epoxide began to crystallize. The mixture was allowed to cool to 25°C over 30 min and 75 mL of methylcyclohexane were added in 10 mL portions with stirring over 30 min. The mixture was aged for 1 h and the crystals were filtered off and washed with 2 x 20 mL methylcyclohexane and dried to give 24.10 g (64%) of the epoxide as a white crystalline solid of 99.9 A% purity by HPLC. The mother liquor and washes were evaporated to an oil and dissolved in 40 mL IPAC. The solution was treated with 10 g of Darco G60 carbon for 2 h at 25°C and filtered through a pad of Solkafloc. The filtrate was evaporated down to ca 20 mL and 40 mL of methylcyclohexane were added. The crystalline epoxide was filtered off and washed with 2 x 10 mL methylcyclohexane to afford another 4.96 g (13%) of epoxide 96.2 A% by HPLC. The conversion of the iodohydrin to epoxide may also be accomplished by the addition of 1.7 M potassium-tert-butoxide in THF (0.70 mL, 1.2 mmol) or 5 M potassium hydroxide in methanol (0.24 mL, 1.2 mmol) or DIEA (155 mg, 1.2 mmol) to a solution of the iodohydrin (505 mg, 1.0 mmol) in IPAC (2-3 mL) followed by washing with 2 x 2 mL water and crystallization from methylcyclohexane-IPAC.

| | |
|---|---|
| Allyl acetonide | 26.15 g |
| N-chlorosuccinamide (NCS) | 22.7 g |
| Sodium iodide | 25.5 g |
| Aqueous sodium bicarbonate (0.5 M) | 350 mL |
| Isopropyl acetate (IPAC) | 300 mL |

The NCS and NaI were stirred together in 200 mL of water for 20 min. The mixture turned dark brown then immediately a black solid separated out. The solid dissolved and the color faded to clear yellow with further aging. The crude allyl acetonide was dissolved in IPAC and stirred with the aqueous sodium bicarbonate and the clear yellow solution prepared above for 17 h. Aqueous sodium bisulfite (38-40%) solution was added and the upper organic phase was separated. The organic phase was washed with 300 mL water and 2 x 100 mL brine. At this point the crude iodohydrin solution in IPAC can be directly taken on to the next step or the solution could be evaporated and crystallized from methylcyclohexane-IPAC to give the iodohydrin as a pale yellow crystalline solid.

### EXAMPLE 4

### Preparation of Amide 1

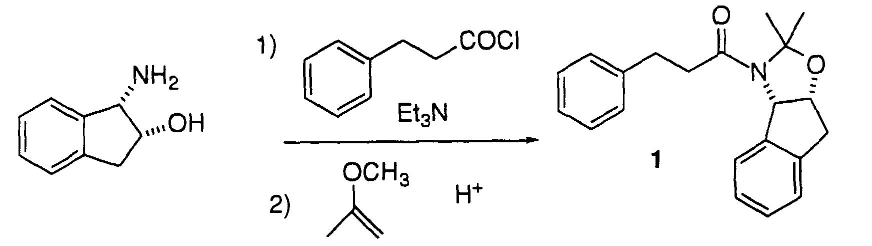

A solution of (-)-cis-1-aminoindan-2-ol (884 g, 5.93 mol) in 17.8 L of dry THF (KF = 55 mg/mL) (KF stands for Karl Fisher titration for water) and triethylamine (868 mL, 6.22 mol) in a 50 L round bottom flask equipped with a thermocouple probe, mechanical stirrer, and a nitrogen inlet adapter and bubbler, was cooled to 15°C. Then, 3-phenylpropionyl chloride (1000 g, 5.93 mol) was added over 75 minutes, while the internal temperature between 14-24°C with an ice-water cooling batch. After addition, the mixture was aged at 18 to 20°C for 30 minutes and checked by HPLC analysis for the disappearance of (-)-cis-1-aminoindan-2-ol.

Progress of the reaction is monitored by high performance liquid chromatography (HPLC) analysis: 25 cm Dupont C8-RX column, 60:40 acetonitrile/10mM (KH₂PO₄/K₂HPO₄), 1.0 mL/min., injection volume = 20 mL, detection = 200 nm, sample preparation = 500 X dilution. Approximate retention times:

| retention time (min. | identity |
|---|---|
| 6.3 | cis-aminoindanol |

The reaction was treated with pyridinium *p*-toluene-sulfonate (241 g, 0.96 mol, 0.16 equiv.) and stirred for 10 minutes (the pH of the mixture after diluting 1 mL sample with an equal volume of water is between 4.3-4.6). Then, 2-methoxypropene (1.27 L, 13.24 mol, 2.2 equiv.) was added and reaction was heated to 38-40°C for 2 h. The reaction mixture was cooled to 20°C and partitioned with ethyl acetate (12 L) and 5% aqueous NaHCO₃ (10 L). The mixture was agitated and the layers were separated. The ethyl acetate extract was washed with 5% aqueous NaHCO₃ (10 L) and water (4 L). The ethyl acetate extract was dried by atmospheric distillation and solvent switched to cyclohexane (total volume of ~30L). At the end of the distillation and concentration (20 volume % of ethyl acetate extraction volume), the hot cyclohexane solution was allowed to slowly cool to 25°C to crystallize the product. The resulting slurry was further cooled to 10°C and aged for 1 h. The product was isolated by filtration and the wet cake was washed with cold (10°C) cyclohexane (2 X 800 mL). The washed cake was dried under vacuum (26" of Hg) at 40°C to afford 1.65 kg of acetonide **1** (86.4%, 98 area% by HPLC), ¹H NMR (300.13 MHz, CDCl₃, major rotamer) δ 7.36-7.14 (m, 9 H), 5.03 (d, J=4.4, 1 H), 4.66 (m, 1 H) 3.15 (m, 2 H), 3.06 (br s, 2 H), 2.97 (m, 2 H), 1.62 (s, 3 H), 1.37 (s, 3 H); ¹³C NMR (75.5 MHz, CDCl₃, major rotamer) δ_{c} 168.8, 140.9, 140.8, 140.6, 128.6, 128.5, 128.4, 127.1, 126.3, 125.8, 124.1, 96.5, 78.6, 65.9, 38.4, 36.2, 31.9, 26.5, 24.1.

| | | | |
|---|---|---|---|
| Anal. Calcd for C₂₁H₂₃NO₂ | C, 78.47; | H, 7.21; | N, 4.36. |
| Found | C, 78.65; | H, 7.24; | N, 4.40. |

### EXAMPLE 5

### Preparation of Epoxide 3 Tosylate Method

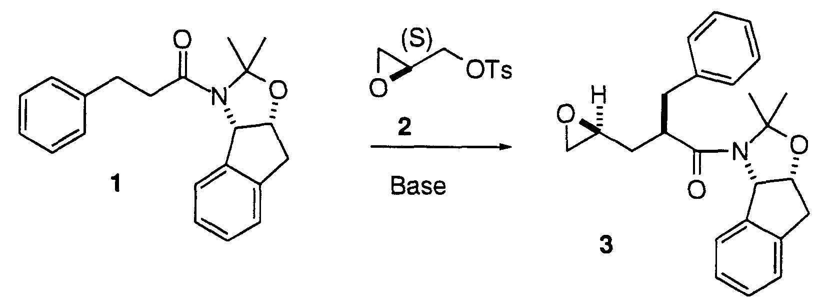

A solution of acetonide **1** (1000 g, 3.11 mol) and 2(S)glycidyl tosylate **2** (853 g, 3.74 mol, 1.2 equiv.) in 15.6 L of THF (KF = 22 mg/mL) in a 50 L 4-neck round bottom flask, equipped with a thermocouple, mechanical stirrer, addition funnel and nitrogen inlet adapter was degassed 3 times via vacuum-nitrogen purge and cooled to -56°C. Then, lithium hexamethyldisilazide (LiN[(CH₃)₃Si]₂)(2.6 L, 1.38 M, 1.15 equiv.) was added over 2 h, while keeping the internal temperature between -50 to -45°C. The reaction mixture was stirred at -45 to -40°C for 1 h and then allowed to warm to -25°C over 1 h. The mixture is stirred between -25 to -22°C for 4 h (or until the starting acetonide is 3.0 area %).

Progress of the reaction is monitored by HPLC analysis: 25 cm X 4.6 nm Zorbax Silica column, 20% ethyl acetate in hexane, 2.0 mL/min, injection volume = 20 mL, detection = 254 nm, sample preparation = 100 X dilution. Approximate retention times:

| retention time (min) | identity |
|---|---|
| 5.5 | amide **1** |
| 6.5 | glycidyl tosylate **2** |
| 13.5 | epoxide **3** |

The reaction mixture was quenched with DI water (6.7 L) at -15°C and partitioned with ethyl acetate (10 L). The mixture was agitated and the layers were separated. The ethyl acetate extract was washed with a mixture of 1 % aqueous NaHCO₃ (5 L) and saturated NaCl (0.5 L). The ethyl acetate extract (28.3 L) was concentrated by vacuum distillation (28" of Hg) and additional ethyl acetate was added to complete the solvent switch to ethyl acetate (final volume = 11.7 L). The ethyl acetate concentrate was further solvent switched to MeOH to crystallize the product and concentrated to a final volume of 3.2 L. The residual ethyl acetate solvent was removed by charging 10 L of methanol and collecting 10 L of distillate. The resulting slurry was stirred at 22°C for 1 h, then cooled to 5°C and aged for 0.5 h. The product was isolated by filtration and the wet cake was washed with cold methanol (2 X 250 mL). The washed cake was dried under vacuum (26" of Hg) at 25°C to afford 727 g of epoxide **3** (61.2%, 98.7 area % of the major epoxide by HPLC): ¹³C NMR (300 MHz, CDCl₃) δ 171.1, 140.6, 140.5, 139.6, 129.6, 128.8, 128.2, 127.2, 126.8, 125.6, 124.1, 96.8, 79.2, 65.8, 50.0, 48.0, 44.8, 39.2, 37.4, 36.2, 26.6, 24.1.

### EXAMPLE 6

### Preparation of penultimate 6

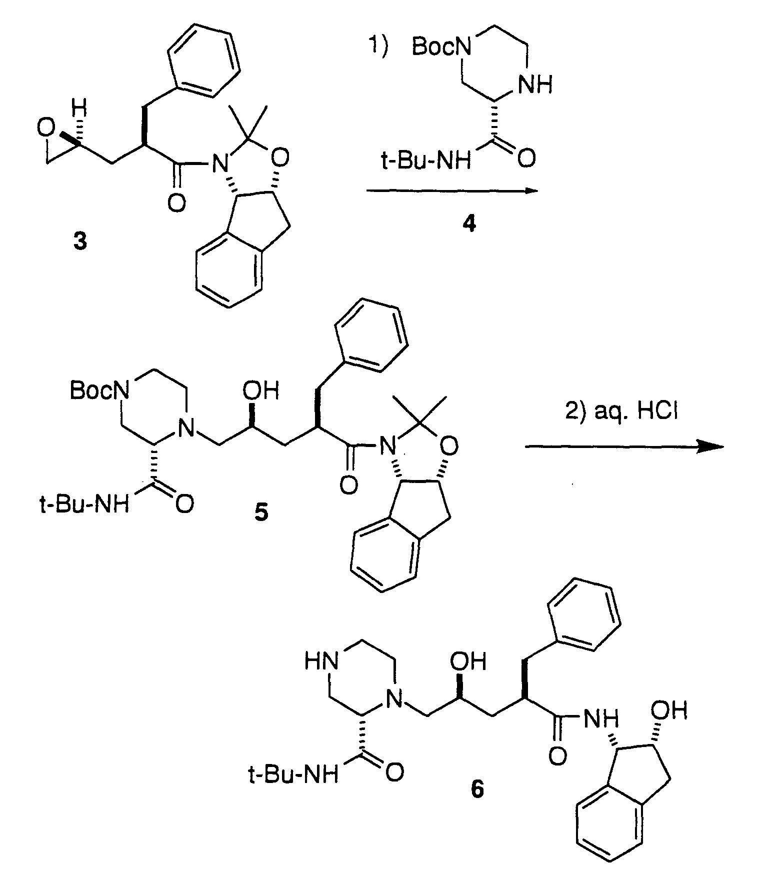

A slurry of the 2(S)-t-butylcarboxamide-4-*N*-Boc-piperazine **4** (1950 g, 6.83 mol, >99.5% ee) (ee = enantiomeric excess) and the epoxide **3** (2456 g, 97.5:2.5 mixture of 4*S/R* epoxides, 6.51 mol) in isopropanol (2-propanol, 18.6 L) in a 72 L round bottom flask with four inlets, equipped with a mechanical stirrer, reflux condenser, steam bath, Teflon coated thermocouple and nitrogen inlet, was heated to reflux (internal temperature was 84-85°C). After 40 min, a homogeneous solution was obtained. The mixture was heated at reflux for 28 h.

The internal temperature during reflux was 84-85°C. Progress of the reaction was monitored by HPLC analysis: 25 cm Dupont C8-RX column, 60:40 acetonitrile/10 mM (KH₂PO₄/K₂HPO₄), 1.0 mL/min., detection = 220 nm, sample preparation = 2 µL, reaction mixture diluted to 1 mL in acetonitrile. Approximate retention times:

| retention time (min) | identity |
|---|---|
| 4.8 | piperazine **4** |
| 8.9 | epoxide **3** |
| 15.2 | coupled product **5** |

After 28 h, the remaining epoxide **3** and coupled product **5** (by HPLC analysis) were 1.5 area % and 91-93 area %, respectively. The mixture was cooled to 0 to 5°C and 20.9 L of 6 N HCl was added while keeping the temperature below 15°C. After the addition was complete, the mixture was warmed to 22°C. Evolution of gas is noted at this point (isobutylene). The mixture was aged at 20 to 22°C for 6 h.

Progress of the reaction was monitored by HPLC analysis: same conditions as above. Approximate retention times:

| retention time (min) | identity |
|---|---|
| 7.0 | cis-aminoindanol |
| 11.9 | penultimate **6** |
| 15.1 | coupled product **5** |

The mixture was cooled to 0°C and 7.5 L of 50% NaOH was slowly added to adjust the pH of the mixture to pH=11.6, while keeping the temperature less than 25°C during the addition. The mixture was partitioned with ethyl acetate (40 L) and water (3 L). The mixture was agitated and the layers were separated. The organic phase (60 L) was concentrated under reduced pressure (29" of Hg) and solvent switched to DMF and concentrated to a final volume of 10.5 L (KF = 1.8 mg/mL). The HPLC assay yield of **6** in ethyl acetate was 86.5%. The penultimate compound **6** in DMF was directly used in the next step without further purification. For isolated **6:** ¹³C NMR (75.4 MHz, CDCl₃) δ 175.2, 170.5, 140.8, 140.5, 139.9, 129.1, 128.5, 127.9, 126.8, 126.5, 125.2, 124.2, 73.0, 66.0, 64.8, 62.2, 57.5, 49.5, 47.9, 46.4, 45.3, 39.6, 39.3, 38.2, 28.9.

### EXAMPLE 7

### Preparation of monohydrate of Compound J

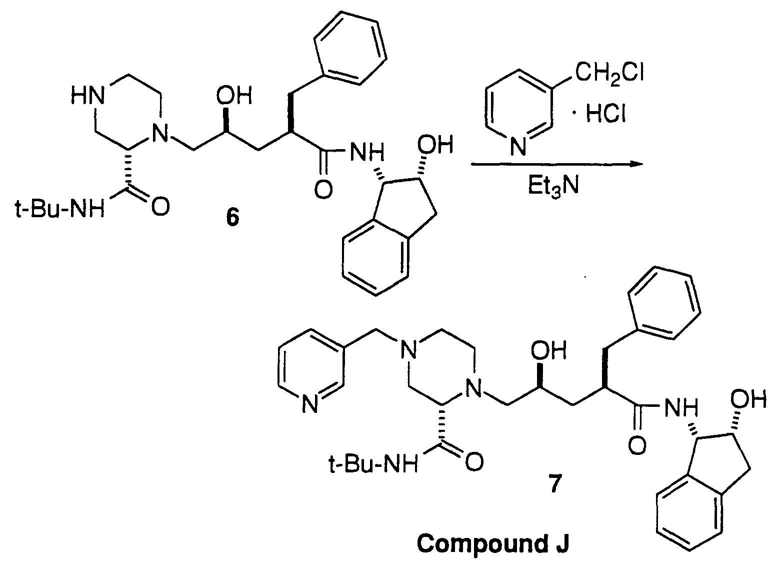

The solution of **6** in DMF (10.5 L, KF = 10 mg/mL) from the previous step was charged with 8 L of sieve dried DMF (KF. < 30 mg/L) and the mixture was heated with a steam bath under vacuum of 30" of Hg to distill off mainly water and/or any residual isopropanol or ethyl acetate solvent. The final concentrate volume was 13.5 L (KF = 1.8 mg/mL) and then triethylamine (2.86 L, 20.51 mol) was added to the 25°C solution followed by 3-picolyl chloride hydrochloride (96%, 1287 g, 7.84 mol). The resulting slurry was heated to 68°C.

The progress of the reaction was followed by HPLC analysis using the same conditions as the previous step. Approximate retention times:

| Retention time (min) | Identity |
|---|---|
| 2.7 | DMF |
| 4.2 | 3-picolyl chloride |
| 4.8 | Compound J |
| 9.1 | penultimate **6** |

The mixture was aged at 68°C until the residual penultimate compound **6** was < 0.3 area % by HPLC analysis.

The mixture was stirred at 68°C for 4 h, then cooled to 25°C and partitioned with ethyl acetate (80 L) and a mixture of 24 L of saturated aqueous NaHCO₃ and distilled water (14 L). The mixture was agitated at 55°C and the layers were separated. The ethyl acetate layer was washed three times with water (20 L) at 55°C. The washed ethyl acetate layer is concentrated at atmospheric pressure to a final pot volume of 30 L. At the end of the atmospheric concentration, water (560 mL) was added to the hot solution and the mixture was cooled to 55°C and seeded with Compound J monohydrate. The mixture was cooled to 4°C and filtered to collect the product. The product was washed with cold ethyl acetate (2 X 3 L), and dried at house vacuum at 25°C to afford 2905 g (70.7%) of Compound J monohydrate as a white solid.

### EXAMPLE 8

### Pyrazine-2-tert-butyl carboxamide 9

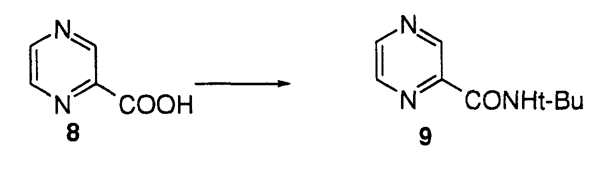

| | |
|---|---|
| 2-Pyrazinecarboxylic acid (**8**) | 3.35 kg (27 mol) |
| Oxalyl chloride | 3.46 kg (27.2 mol) |
| tert-Butylamine (KF = 460 µg/ml) | 9.36 L (89 mol) |
| EtOAc (KF = 56 µg/ml) | 27 L |
| DMF | 120 mL |
| 1-Propanol | 30 L |

The carboxylic acid **8** was suspended in 27 L of EtOAc and 120 mL of DMF in a 72 L 3-neck flask with mechanical stirring under N₂ and the suspension was cooled to 2°C. The oxalyl chloride was added, maintaining the temperature between 5 and 8°C.

The addition was completed in 5h. During the exothermic addition CO and CO₂ were evolved. The HCl that was formed remained largely in solution. A precipitate was present which is probably the HCl salt of the pyrazine acid chloride. Assay of the acid chloride formation was carried out by quenching an anhydrous sample of the reaction with t-butylamine. At completion <0.7% of acid **8** remained.

The assay for completion of the acid chloride formation is important because incomplete reaction leads to formation of a bis-tert-butyl oxamide impurity.

The reaction can be monitored by HPLC: 25 cm Dupont Zorbax RXC8 column with 1 mL/min flow and detection at 250 nm; linear gradient from 98% of 0.1% aqueous H₃PO₄ and 2% CH₃CN to 50% aqueous H₃PO₄ and 50% CH₃CN at 30 min. Retention times: acid **8** = 10.7 min, amide **9** = 28.1 min.

The reaction mixture was aged at 5°C for 1 h. The resulting slurry was cooled to 0°C and the tert-butylamine was added at such a rate as to keep the internal temperature below 20°C.

The addition required 6 h, as the reaction was very exothermic. A small portion of the generated tert-butylammonium hydrochloride was swept out of the reaction as a fluffy white solid.

The mixture was aged at 18°C for an additional 30 min. The precipitated ammonium salts were removed by filtration. The filter cake was washed with 12 L of EtOAc. The combined organic phases were washed with 6 L of a 3% NaHCO₃ and 2 X 2 L of saturated aq. NaCl. The organic phase was treated with 200 g of Darco G60 carbon and filtered through Solka Flok and the cake was washed with 4 L of EtOAc.

Carbon treatment efficiently removed some purple color in the product.

The EtOAc solution of **9** was concentrated at 10 mbar to 25% of the original volume. 30 L of 1-propanol were added, and the distillation was continued until a final volume of 20 L was reached.

At this point, the EtOAc was below the limit of detection in the ¹H NMR (<1%). The internal temperature in this solvent change was <30°C. A 1-propanol/EtOAC solution of 3 was stable to reflux atatmospheric pressure for several days.
Evaporation of an aliquot gave a tan solid m.p 87-88°C. ¹³C NMR (75 MHz, CDCl₃, ppm) 161.8, 146.8, 145.0, 143.8, 142.1, 51.0, 28.5.

### EXAMPLE 9

### rac-2-tert-Butyl-carboxamide-piperazine 10

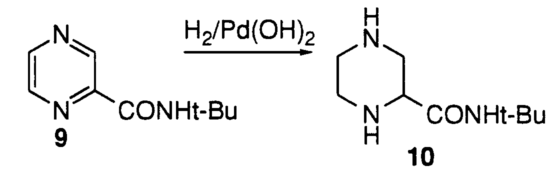

### Materials

Pyrazine-2-tert-butylcarboxamide **9** 2.4 kg (13.4 mol) in 1-Propanol solution 12 L 20% Pd(OH)₂/C 16 wt.% water 144 g

The pyrazine-2-tert-butylcarboxamide **9**/1-propanol solution was placed into the 5 gal autoclave. The catalyst was added and the mixture was hydrogenated at 65°C at 40 psi (3 atm) of H₂.

After 24 h the reaction had taken up the theoretical amount of hydrogen and GC indicated <1% of **9.** The mixture was cooled, purged with N₂ and the catalyst was removed by filtration through Solka Floc. The catalyst was washed with 2 L of warm 1-propanol.

It was found that the use of warm 1-propanol during washing of the filter cake improved filtration and lowered the losses of product on the filter cake.

The reaction was monitored by GC: 30 m Megabore column, from 100°C to 160°C at 10°C/min, hold 5 min, then at 10°C/min to 250°C, retention times: **9** = 7.0 min, **10** = 9.4 min. The reaction could also be monitored by TLC with EtOAc/MeOH (50:50) as solvent and Ninhydrin as developing agent.

Evaporation of an aliquot indicated that the yield over amidation and hydrogenation is 88% and that the concentration of **10** is 133 g/L.

Evaporation of an aliquot gave **10** as a white solid m.p. 150-151°C; ¹³C NMR (75 MHz, D₂O, ppm) 173.5, 59.8, 52.0, 48.7, 45.0, 44.8, 28.7.

### EXAMPLE 10

### (S)-2-tert-Butyl-carboxamide-piperazine bis (S)-Camphorsulfonic acid salt (S)-11

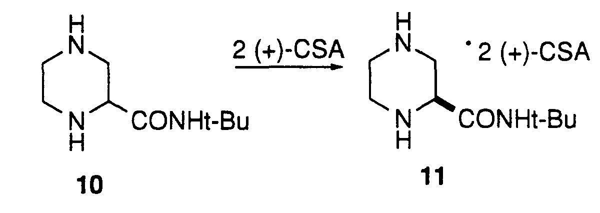

| Materials | |
|---|---|
| rac-2-tert-Butyl-carboxamide-piperazine **10** | 4.10 kg (22.12 mol) |
| in 1-Propanol Solution | in 25.5 Kg solvent |
| (S)-(+)-10-Camphorsulfonic acid | 10.0 Kg (43.2 mol) |
| 1-Propanol | 12 L |
| Acetonitrile | 39 L |
| Water | 2.4 L |

The solution of amine **10** in 1-propanol was charged to a 100 L flask with an attached batch concentrator. The solution was concentrated at 10 mbar and a temperature < 25°C to a volume of ca 12 L.

At this point the product had precipitated from the solution, but went back into a solution when the mixture was heated to 50°C.

Analysis of a homogeneous aliquot indicated that the concentration of **10** was 341 g/L The concentration was determined by HPLC: 25 cm Dupont Zorbax RXC8 column with 1.5 mL/min flow and detection at 210 nm, isocratic (98/2) CH₃CN/0.1% aqueous H₃PO₄. Retention time of **10**:2.5 min.

Acetonitrile (39 L) and water (2.4 L) were added to give a clear, slightly brown solution.

Determination of the water content by KF titration and CH₃CN/1-propanol ratio by ¹H NMR integration showed that the CH3CN/1-propanol/H₂O ratio was 26/8/1.6. The concentration in the solution was 72.2 g/L.

The (S)-10-camphorsulfonic acid was charged over 30 min in 4 portions at 20°C. The temperature rose to 40°C after the CSA was added. After a few minutes a thick white precipitate formed. The white slurry was heated to 76°C to dissolve all the solids, the slightly brown solution was then allowed to cool to 21°C over 8 h.

The product precipitated at 62°C. The product was filtered without aging at 21°C, and the filter cake was washed with 5 L of the CH₃CN/1-propanol/H₂O 26/8/1.6 solvent mixture. It was dried at 35°C in the vacuum oven with N₂ bleed to give 5.6 Kg (39%) of **11** as a white crystalline solid m.p. 288-290°C (with decomp.) [α]_{D}²⁵ = 18.9° (c = 0.37, H₂O). ¹³C NMR (75 MHz, D₂O, ppm) 222.0, 164.0, 59.3, 54.9, 53.3, 49.0, 48.1, 43.6, 43.5, 43.1, 40.6, 40.4, 28.5, 27.2, 25.4, 19.9, 19.8.

The ee of the material was 95% according to the following chiral HPLC assay: an aliquot of **11** (33 mg) was suspended in 4 mL of EtOH and 1 mL of Et₃N. Boc₂O (11 mg) was added and the reaction mixture was allowed to age for 1 h. The solvent was completely removed *in vacuo*, and the residue was dissolved in ca. 1 mL of EtOAc and filtered through a Pasteur pipet with SiO₂, using EtOAc as eluent. The evaporated product fractions were redissolved in hexanes at ca. 1 mg/mL. The enantiomers were separated on a Daicel Chiracell AS column with a hexane/IPA (97:3) solvent system at a flow rate of 1 mL/min and detection at 228 nm. Retention times: S antipode = 7.4 min, R = 9.7 min.

### EXAMPLE 11

### (S)-2-tert-Butylcarboxamide-4-tert-butoxycarbonyl-piperazine 4 from salt 11

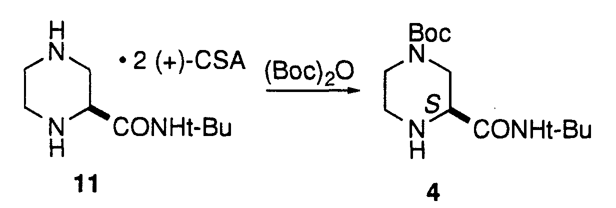

| Materials | |
|---|---|
| (S)-2-tert-Butyl-carboxamide-piperazine | |
| Bis (S) - (+) - CSA salt **11**, 95% ee | 5.54 Kg (8.53 mol) |
| Di-tert-butyl dicarbonate | 1.86 Kg (8.53 mol) |
| Et₃N | 5.95L (42.6 mol) |
| EtOH Punctilious 200 proof | 55 L |
| EtOAc | 2 L |

To the (S)-CSA salt **11** in a 100 L 3-neck flask with an addition funnel under N₂ was added EtOH, followed by triethylamine at 25°C. The solid dissolved readily on the addition of the Et₃N. The Boc₂O was dissolved in EtOAc and charged to the addition funnel. The solution of Boc₂O in EtOAc was added at such a rate as to keep the temperature below 25°C. The addition took 3 h. The reaction mixture was aged for 1 h after completion of the addition of the Boc₂O solution.

The reaction can be monitored by HPLC:25 cm Dupont Zorbax RXC8 column with 1 mL/min flow and detection at 228 nm, isocratic (50/50) CH₃CN/0.1 M KH₂PO₄ adjusted to pH=6.8 with NaOH. Retention time of **4** = 7.2 min. The chiral assay was carried out using the same system as in the previous step. The reaction could also be monitored by TLC with a 100% EtOAc as the solvent. (R_{f}=0.7)

The solution was then concentrated to ca. 10 L at an internal temperature of <20°C in a batch-type concentrator under 10 mbar vacuum. The solvent switch was completed by slowly bleeding in 20 L of EtOAc and reconcentrating to ca 10 L. The reaction mixture was washed into an extractor with 60 L of EtOAc. The organic phase was washed with 16 L of 5% aqueous Na₂CO₃ solution, 2 X 10 L Di water and 2 X 6 L of saturated aqueous sodium chloride. The combined aqueous washes were back extracted with 20 L of EtOAc and the organic phase was washed with 2 X 3 L water and 2 X 4 L of saturated aqueous sodium chloride. The combined EtOAc extracts were concentrated under 10 mbar vacuum with an internal temperature of <20°C in a 100 L batch-type concentrator to ca. 8 L. The solvent switch to cyclohexane was achieved by slowly bleeding in ca. 20 L of cyclohexane, and reconcentrating to ca. 8 L. To the slurry was added 5 L of cyclohexane and 280 mL of EtOAc and the mixture was heated to reflux, when everything went into solution. The solution was cooled and seed (10 g) was added at 58°C. The slurry was cooled to 22°C in 4 h and the product was isolated by filtration after a 1 h age at 22°C. The filter cake was washed with 1.8 L of cyclohexane and dried in the vacuum oven at 35°C under N₂ bleed to give 1.87 Kg (77%, >99.9 area % by HPLC, R-isomer below level of detection) of **4** as a slightly tan powder. [α]_{D}²⁵ = 22.0° (c = 0.20, MeOH), m.p. 107°C; ¹³C NMR (75 MHz, CDCl₃, ppm) 170.1, 154.5, 79.8, 58.7, 50.6, 46.6, 43.6, 43.4, 28.6, 28.3.

While the foregoing specification teaches the principles of the present invention, with examples provided for the purpose of illustration, it will be understood that the practice of the invention encompasses all of the usual variations, adaptations, modifications, deletions or additions of procedures and protocols described herein, as come within the scope of the following claims and it equivalents.

## Claims

1. A process of synthesizing the epoxide of Formula I, 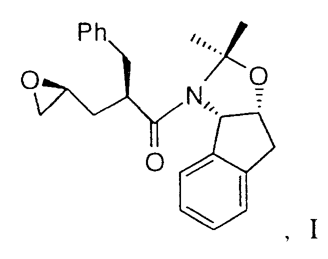 comprising the steps of:
(a) contacting one equivalent of the allyl acetonide of Formula II, 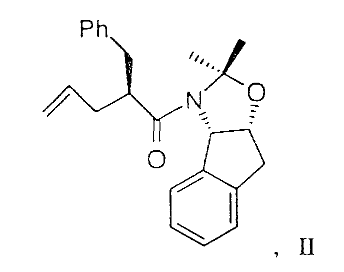 with one to two equivalents of a halogenating agent in solvent mixed with aqueous weak base, at a temperature range of between -40°C and 100°C, to form the halohydrin of Formula III, and 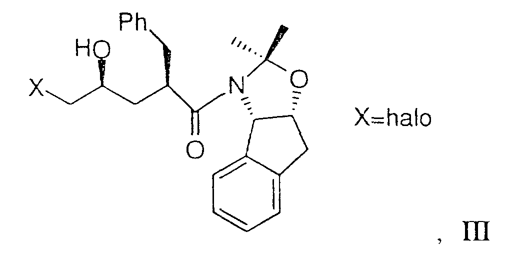
(b) adding base in solvent or solvents to elicit formation of the epoxide of Formula I.

2. The process of claim 1 further comprising
reacting one equivalent of the acetonide 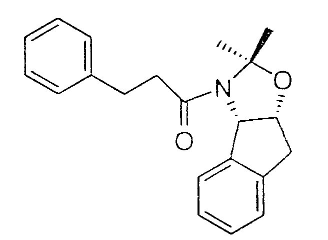 with one equivalent of allylhalide in strong base, to give the allyl acetonide of Formula II.

3. The process of Claim 1 or 2, comprising the steps of:
(a) contacting one equivalent of the allyl acetonide of Formula II, 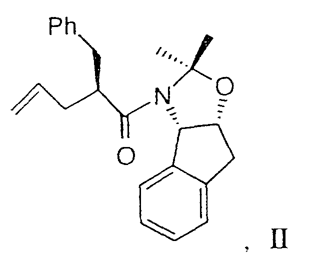 with one to two equivalents of a halogenating agent, in solvent mixed with aqueous weak base, at a temperature range of between -40°C and 100°C, to form the halohydrin of Formula III, 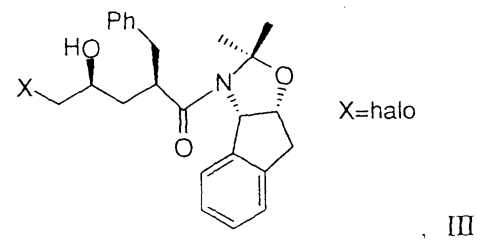 said halogenating agent selected from the group consisting of N-iodosuccinimide, N-bromosuccinimide or N-chlorosuccinimide, the last two of which may be combined with an iodide salt, said solvent selected from dichloromethane, isopropyl acetate (IPAC), ethyl acetate (EtOAc), dimethoxyethane (DME), and methyl t-butyl ether (MTBE), said weak base selected from basic alumina or sodium bicarbonate, and
(b) adding base in water to elicit formation of the epoxide of Formula I, said base selected from lithium hydroxide, sodium hydroxide, potassium hydroxide, tetralkylammonium hydroxide; any C₁₋₄ alkoxide of lithium, sodium or potassium; and diisopropylethylamine (DIEA).

4. The process of Claim 1, 2 or 3, comprising
reacting one equivalent of the acetonide 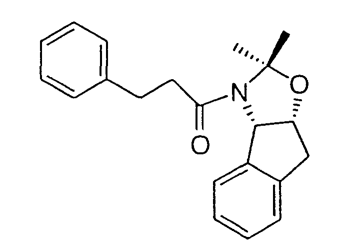 with one equivalent of allylhalide in strong base, said allyl halide selected from allyl chloride, allyl bromide and allyl iodide, to give the allyl acetonide of Formula II.

5. A process of synthesizing the epoxide of Formula I, 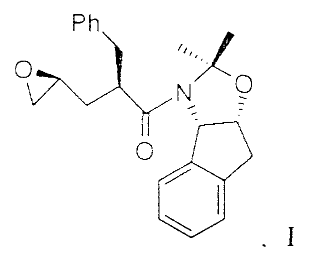 comprising the steps of:
(a) contacting one equivalent of the allyl acetonide of Formula II, 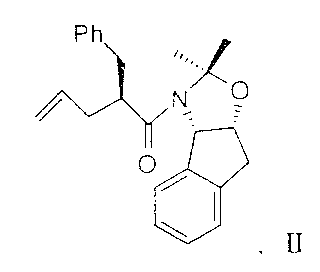 said allyl acetonide dissolved in isopropyl acetate, with one to two equivalents of N-iodosuccinimide in about 0.5 M aqueous sodium bicarbonate, at room temperature, to form the iodohydrin of Formula III, and 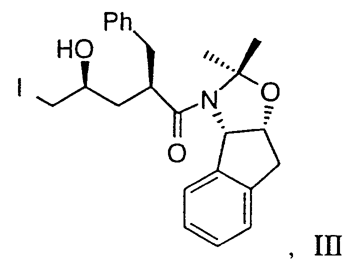
(b) adding alkali hydoxide in water to elicit formation of the epoxide of Formula **I**.

6. A process of synthesizing the epoxide of Formula **I**, 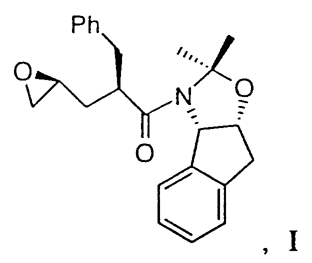 comprising the steps of:
(a) reacting one equivalent of the acetonide 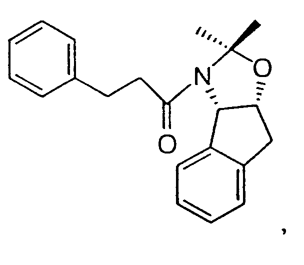 said acetonide dissolved in ethereal solvent, with one equivalent of allylbromide and one equivalent of about 1.0-2.0 M lithiumhexamethyldisilazide (in ethereal solvent), to give the allyl acetonide of Formula **II**, 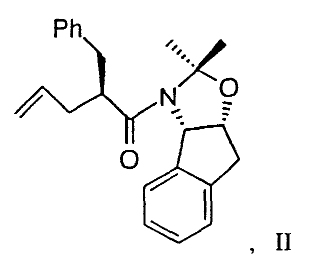
(b) mixing thereto one to two equivalents of N-iodosuccinimide in about 0.5 M aqueous sodium bicarbonate, at room temperature, to form the iodohydrin of Formula **III**, and 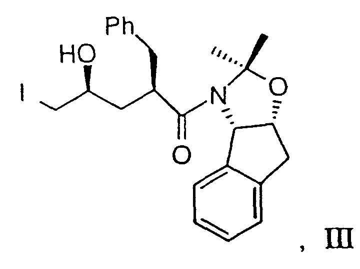
(c) adding alkali hydroxide in water to elicit formation of the epoxide of Formula **I**.

## Patentansprüche

1. Ein Verfahren zur Synthese des Epoxids der Formel I 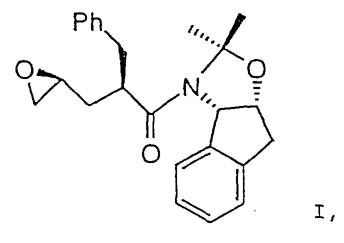 umfassend die Schritte:
(a) Inkontaktbringen eines Äquivalents des Allylacetonids der Formel II 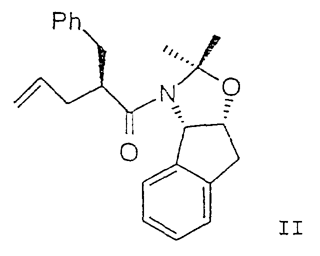 mit ein bis zwei Äquivalenten eines Halogenierungsmittels in einem mit wäßriger schwacher Base vermischten Lösungsmittel in einem Temperaturbereich zwischen -40°C und 100°C, um das Halogenhydrin der Formel III zu bilden 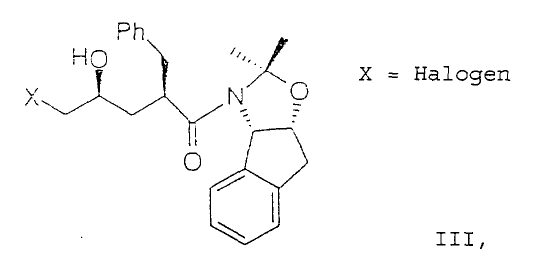 und
(b) Zugabe einer Base in Lösungsmittel oder Lösungsmitteln, um die Bildung des Epoxids der Formel I zu bewirken.

2. Das Verfahren nach Anspruch 1, das ferner umfaßt die Umsetzung eines Äquivalents des Acetonids 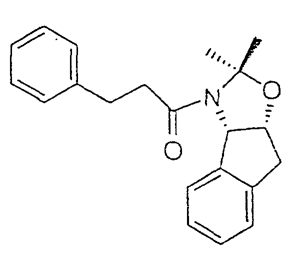 mit einem Äquivalent Allylhalogenid in starker Base, um das Allylacetonid der Formel II zu ergeben.

3. Das Verfahren nach Anspruch 1 oder 2, umfassend die Schritte:
(a) Inkontaktbringen eines Äquivalents des Allylacetonids der Formel II 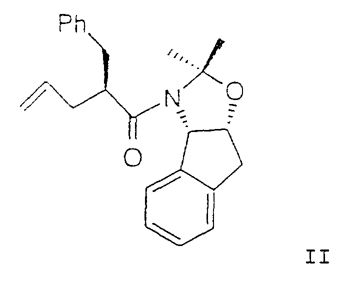 mit ein bis zwei Äquivalenten eines Halogenierungsmittels in einem mit wäßriger schwacher Base vermischten Lösungsmittel in einem Temperaturbereich zwischen -40°C und 100°C, um das Halogenhydrin der Formel III zu bilden 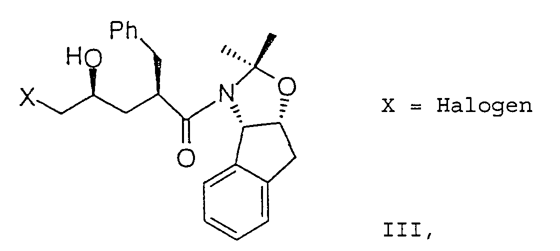 wobei das Halogenierungsmittel ausgewählt ist aus der Gruppe, bestehend aus N-Iodsuccinimid, N-Bromsuccinimid oder N-Chlorsuccinimid, wobei die letzten beiden mit einem Iodidsalz kombiniert werden können, das Lösungsmittel ausgewählt ist aus Dichlormethan, Isopropylacetat (IPAC), Ethylacetat (EtOAc), Dimethoxyethan (DME) und Methyl-t-butylether (MTBE), die schwache Base ausgewählt ist aus basischem Aluminiumoxid oder Natriumhydrogencarbonat, und
(b) Zugabe von Base in Wasser, um die Bildung des Epoxids der Formel I zu bewirken, wobei die Base ausgewählt ist aus Lithiumhydroxid, Natriumhydroxid, Kaliumhydroxid, Tetraalkylammoniumhydroxid, einem beliebigen C₁₋₄-Alkoxid von Lithium, Natrium oder Kalium und Diisopropylethylamin (DIEA).

4. Das Verfahren nach Anspruch 1, 2 oder 3, umfassend die Umsetzung eines Äquivalents des Acetonids 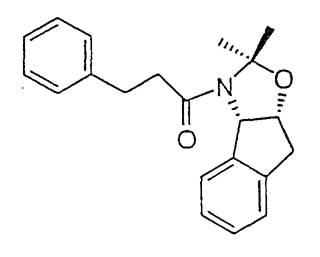 mit einem Äquivalent Allylhalogenid in starker Base, wobei das Allylhalogenid ausgewählt ist aus Allylchlorid, Allylbromid und Allyliodid, um das Allylacetonid der Formel II zu ergeben.

5. Ein Verfahren zur Synthese des Epoxids der Formel I 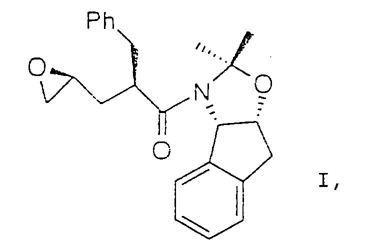 umfassend die Schritte:
(a) Umsetzung eines Äquivalents des Allylacetonids der Formel II 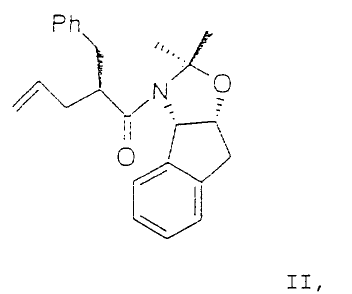 wobei das Allylacetonid in Isopropylacetat gelöst ist, mit einem oder zwei Äquivalenten N-Iodsuccinimid in 0,5M wäßrigem Natriumhydrogencarbonat bei Raumtemperatur, um das Iodhydrin der Formel III zu bilden 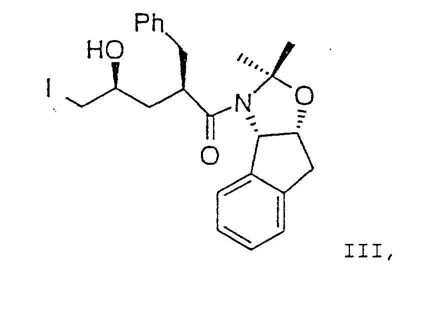 und
(b) Zugabe von Alkalihydroxid in Wasser, um die Bildung des Epoxids der Formel I zu bewirken.

6. Ein Verfahren zur Synthese des Epoxids der Formel I 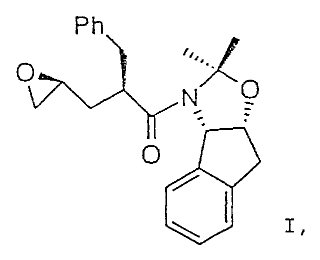 umfassend die Schritte:
(a) Umsetzung eines Äquivalents des Acetonids 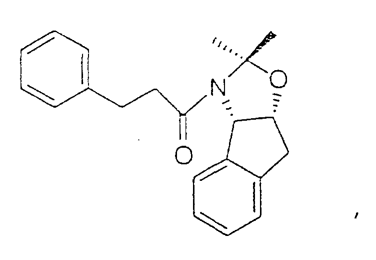 wobei das Acetonid in etherischem Lösungsmittel gelöst ist, mit einem Äquivalent Allylbromid und einem Äquivalent 1,0-2,0M Lithiumhexamethyldisilazid (in etherischem Lösungsmittel), um das Allylacetonid der Formel II zu ergeben 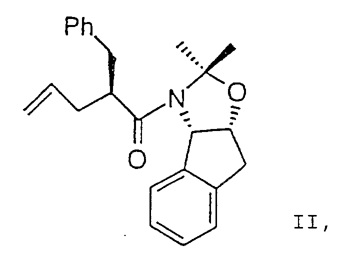
(b) Zumischen von ein bis zwei Äquivalenten N-Iodsuccinimid in 0,5M wäßrigem Natriumhydrogencarbonat bei Raumtemperatur, um das Iodhydrin der Formel III zu bilden 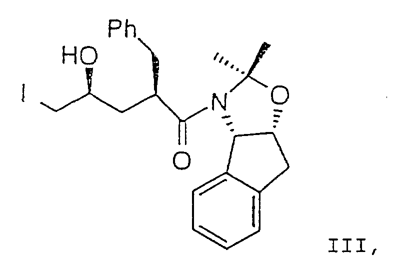 und
(c) Zugabe von Alkalihydroxid in Wasser, um die Bildung des Epoxids der Formel I zu bewirken.

## Revendications

1. Procédé de synthèse de l'époxyde de formule **I**, 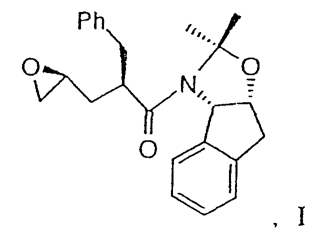 comprenant les étapes consistant à :
(a) mettre en contact un équivalent de l'allylacétonide de formule **II** 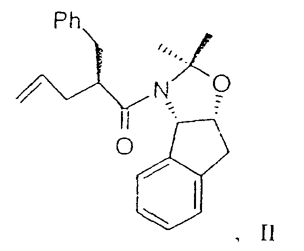 avec un jusqu'à deux équivalents d'un agent d'halogénation dans un solvant mélangé avec une base faible aqueuse, à une température comprise entre -40°C et 100°C pour former l'halogénohydrine de formule **III**, et 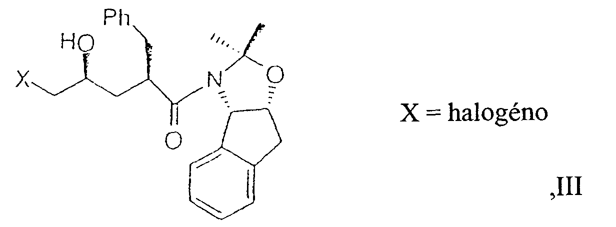
(b) ajouter une base dans un solvant ou des solvants pour permettre la formation de l'époxyde de formule **I**.

2. Procédé selon la revendication 1 comprenant en outre la réaction d'un équivalent de l'acétonide 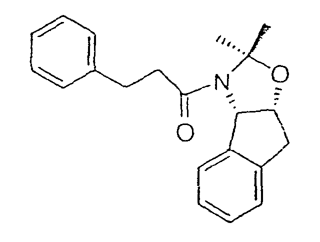 avec un équivalent d'halogénure d'allyle dans une base forte, pour donner l'allylacétonide de formule **II**.

3. Procédé selon la revendication 1 ou 2 comprenant les étapes consistant à
(a) mettre en contact un équivalent de l'allylacétonide de formule **II** 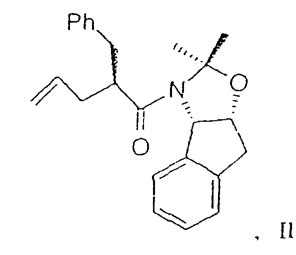 avec un jusqu'à deux équivalents d'un agent d'halogénation dans un solvant mélangé avec une base faible aqueuse, à une température comprise entre -40°C et 100°C pour former l'halogénohydrine de formule **III**, 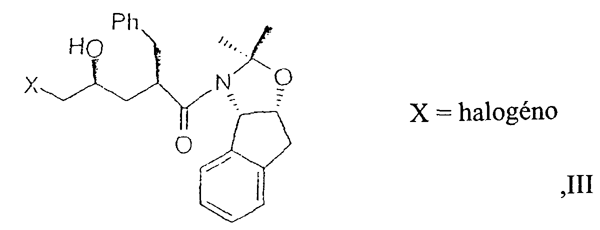 ledit agent d'halogénation étant choisi dans le groupe constitué par le N-iodosuccinimide, le N-bromosuccinimide ou le N-chlorosuccinimide, les deux derniers pouvant être combinés avec un sel d'iodure, ledit solvant étant choisi parmi le dichlorométhane, l'acétate d'isopropyle (IPAC), l'acétate d'éthyle (EtOAc), le diméthoxyéthane (DME) et l'éther de méthyle et de t-butyle (MTBE), ladite base faible étant choisie parmi l'alumine basique et le bicarbonate de sodium, et
(b) ajouter une base dans de l'eau pour permettre la formation de l'époxyde de formule I, ladite base étant choisie parmi l'hydroxyde de lithium, l'hydroxyde de sodium, l'hydroxyde de potassium, l'hydroxyde de tétraalkylammonium ; n'importe quel alcoxyde en C₁₋₄ de lithium, sodium ou potassium ; et la diisopropyléthylamine (DIEA).

4. Procédé selon la revendication 1, 2 ou 3 comprenant la réaction d'un équivalent de l'acétonide 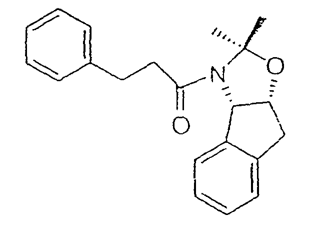 avec un équivalent d'halogénure d'allyle dans une base forte, ledit halogénure d'allyle étant choisi parmi le chlorure d'allyle, le bromure d'allyle et l'iodure d'allyle, pour donner l'allylacétonide de formule **II**.

5. Procédé de synthèse de l'époxyde de formule **I**, 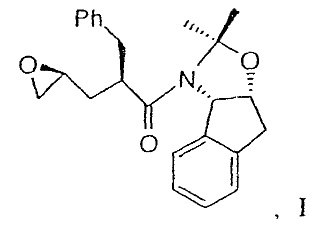 comprenant les étapes consistant à
(a) mettre en contact un équivalent de l'allylacétonide de formule **II** 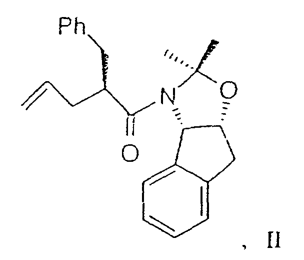 ledit allylacétonide étant dissous dans l'acétate d'isopropyle, avec un jusqu'à deux équivalents de N-iodosuccinimide dans du bicarbonate de sodium aqueux 0,5 M, à la température ambiante, pour former l'iodhydrine de formule **III**, et 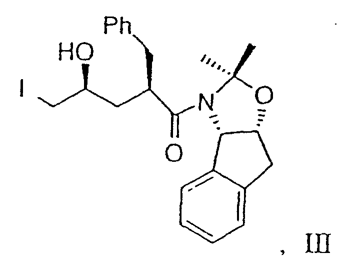
(b) ajouter un hydroxyde de métal alcalin dans l'eau pour permettre la formation de l'époxyde de formule **I**.

6. Procédé de synthèse de l'époxyde de formule **I**, 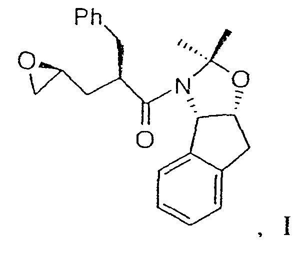 comprenant les étapes consistant à :
(a) faire réagir un équivalent de l'acétonide 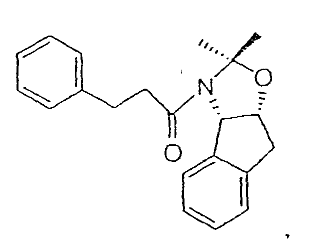 ledit acétonide étant dissous dans un solvant éthéré, avec un équivalent de bromure d'allyle et un équivalent d'hexaméthyldisilazoture de lithium 1,0 - 2,0 M (dans un solvant éthéré), pour donner l'allylacétonide de formule (**II**), 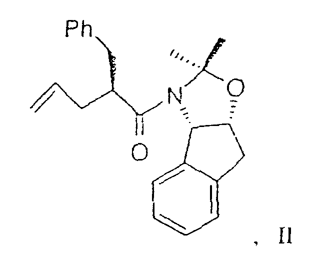
(b) mélanger celui-ci avec un jusqu'à deux équivalents de N-iodosuccinimide dans du bicarbonate de sodium aqueux 0,5 M, à la température ambiante, pour former l'iodhydrine de formule **III**, et 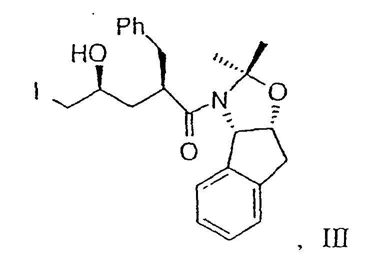
(c) ajouter un hydroxyde de métal alcalin dans l'eau pour permettre la formation de l'époxyde de formule **I**.
